# EUROPEAN PATENT APPLICATION

(11) **EP 1 779 864 A1**
(43) Date of publication of application: **02.05.2007**
(21) Application number: 05760128.8
(22) Date of filing: 14.07.2005
(51) Int. Cl.: A61K 38/22, A61K 9/08, A61P 19/08, A61P 19/10

(54) **PTH-CONTAINING PREPARATION FOR TRANSMUCOSAL ADMINISTRATION**

(30) Priority: 14.07.2004 JP 2004207733
(71) Applicant: CHUGAI SEIYAKU KABUSHIKI KAISHA, Tokyo, 115-8543 (JP)
(72) Inventor: SATO, Katsuhiko, Chugai Seiyaku Kabushiki Kaisha, Chuo-ku, Tokyo 1048301 (JP); SHIMIZU, Masaru, Chugai Seiyaku Kabushiki Kaisha, Gotenba-shi, Shizuoka 4128513 (JP)
(74) Representative: Lloyd, John Scott
(86) International application number: PCT/JP2005/013057
(87) International publication number: WO 2006/006674

(57) **Abstract**

There is provided a method for inhibiting symptoms such as nausea at administration of PTH, and a pharmaceutical composition capable of inhibiting the same. The pharmaceutical composition for transmucosal administration contains hPTH or a derivative thereof and the composition is administered for purpose of increasing bone mass or bone density, and reducing an increase of risk of developing one or more symptoms selected from the group consisting of leg cramps, nausea, headache, and dizziness associated with hPTH administration. The pharmaceutical composition maintains both bone formation action and bone resorption inhibiting action.

## Description

### Technical Field

The present invention relates to a pharmaceutical composition for transmucosal administration containing as an active ingredient human parathyroid hormone (hPTH) or an hPTH derivative, and to a pharmaceutical composition capable of inhibiting a symptom such as nausea which occurs upon administration of hPTH.

### Background Art

Parathyroid hormone (PTH) is known as one of the important hormones involved in bone metabolism. In the past, many reports have been made on actions of PTH on bone.

Osteoporosis is a clinical condition in which a low bone mass and changes in micro structure of bone tissue lead to brittle bone and easily cause fracture. It has been reported that fractures of spines, femoral neck, radius, distal part, etc. associated with osteoporosis lead to a lower QOL, and in particular, femur fracture may be a cause for making a patient bedridden. Therefore, a countermeasure against osteoporosis is desired.

Various factors are intricately involved in fractures, and among these, low bone mineral density (low bone mass) is ranked as a major factor. Therefore, the significance of drug treatment on osteoporosis is to inhibit a bone mass decrease and increase bone mass on a high risk patient that is considered to have a high possibility to cause fracture due to a decrease of bone mass, resulting in the prevention of incident fracture.

At present, commercially available therapeutic drugs for osteoporosis include an estrogen formulation, a calcitonin formulation, a formulation containing an active form of vitamin D, an ipriflavone formulation, a vitamin K formulation, a bisphosphonate formulation, and a calcium formulation. However, most of these drugs are bone resorption inhibitors that inhibit facilitating bone resorption, and thereby exhibit bone mass increasing effect. Thus, they are not bone formation agents that actively work on bone formation and have an action for increasing bone mass.

Human parathyroid hormone (hPTH) is composed of a sequence of 84 amino acids and is a calcium-regulating hormone secreted from the parathyroid glands in response to blood calcium levels. It has been reported that the main body for physiological activity of hPTH is present in 34 fragments (PTH1-34) from the N-terminus, which are involved in binding to a receptor.

Also, it has been reported through basic studies and clinical studies that the effects of hPTH on bone is bone resorption promotion in the case of continuous administration and bone formation promotion in the case of intermittent administration. Thus, hPTH is considered a drug having an action mechanism different from existing therapeutic drugs for osteoporosis, so it is a promising therapeutic drug for osteoporosis having a new mechanism.

However, there have been reports that administration of hPTH to patients caused nausea (bout of vomiting, vomiting, gastric distress, etc.), leg cramps, headache, and dizziness at a certain ratio (see non-patent documents 1 and 2). In order to prevent those symptoms, a dose of hPTH for administration to a patient may be reduced. In that case, bone mass increase action of hPTH, that is an original purpose, may also disappear.

To reduce symptoms at hPTH administration, combination of an antiemetic drug such as teprenone was reported (see patent document 1). However, that method can reduce only bout of vomiting, vomiting, gastric distress, etc. among various symptoms, and administration of one more drug was disadvantageous.

Transnasal pharmaceutical compositions containing hPTH have been reported (see patent documents 2 to 4), but there were no descriptions on the relations with the above symptoms.

Accordingly, there has been a demand for an hPTH administration method for inhibiting a symptom such as nausea, leg cramps, headache, and dizziness while maintaining bone mass increase action of hPTH.
Patent Document 1: JP Patent Publication (Kokai) No. 2003-95974 A
Patent Document 2: JP Patent Publication (Kokai) No. 61-282320 A (1986)
Patent Document 3: JP Patent Publication (Kokai) No. 4-247034 A (1992)
Patent Document 4: International Publication No. WO 02/021136 pamphlet
Non-Patent Document 1: Neer et al., N. Eng. J. Med. 344, 1434-1441, (2001)
Non-Patent Document 2: Fujita et al., Osteoporosis Int. 9. 296-306, (1999)

### Disclosure of the Invention

The present invention has an object of providing a method for inhibiting a symptom such as nausea at administration of the above PTH, and a pharmaceutical composition capable of inhibiting the same.

The present inventors have found that though subcutaneous administration of hPTH as an injection causes symptoms such as nausea, leg cramps, headache, and dizziness, transnasal administration of hPTH leads to no onset or reduces a ratio of onset of those symptoms while maintaining hPTH actions.

Therefore, hPTH may be administered through transmucosal administration such as transnasal administration in order to prevent a symptom such as nausea, leg cramps, headache, and dizziness while maintaining bone mass increase action of hPTH.

Namely, the present invention is as follows.
[1] A pharmaceutical composition for transmucosal administration contains hPTH or a derivative thereof.
[2] The pharmaceutical composition for transmucosal administration of [1], wherein the composition is a bone mass increasing agent.
[3] The pharmaceutical composition for transmucosal administration of [1], wherein the composition is a bone density increasing agent.
[4] The pharmaceutical composition for transmucosal administration of [1], wherein the composition is a therapeutic agent for osteoporosis.
[5] The pharmaceutical composition for transmucosal administration of [1], wherein the composition is a bone resorption inhibitor.
[6] The pharmaceutical composition of any of [1] to [5], wherein the composition accelerates bone formation and inhibits bone resorption.
[7] The pharmaceutical composition of any of [1] to [6], wherein the composition is a transnasal administration agent.
[8] The pharmaceutical composition of any of [1] to [7], wherein hPTH is hPTH1-34.
[9] The pharmaceutical composition of any of [1] to [8], wherein a dose of the composition per day is 250 µg to 1,000 µg.
[10] The pharmaceutical composition of any of [1] to [8], wherein the composition is formulated such that a dose of the composition per day is 250 µg to 1,000 µg.
[11] A pharmaceutical composition for transmucosal administration containing hPTH or a derivative thereof, wherein the composition is administered for purposes of increasing bone mass or bone density, and inhibiting an increase of risk of developing one or more symptoms selected from the group consisting of leg cramps, nausea, headache, and dizziness associated with hPTH administration.
[12] A pharmaceutical composition for transmucosal administration containing hPTH or a derivative thereof, wherein the composition is administered for purposes of increasing bone mass or bone density, and inhibiting an increase of probability of developing one or more symptoms selected from the group consisting of leg cramps, nausea, headache, and dizziness associated with hPTH administration.
[13] A pharmaceutical composition for transmucosal administration containing hPTH or a derivative thereof, wherein the composition is administered for purposes of increasing bone mass or bone density, and preventing a high frequency of developing one or more symptoms selected from the group consisting of leg cramps, nausea, headache, and dizziness associated with hPTH administration.
[14] A pharmaceutical composition for transmucosal administration containing hPTH or a derivative thereof, wherein the composition is administered for purposes of maintaining an action of hPTH for bone mass increase or bone density increase, and reducing a risk of developing one or more symptoms selected from the group consisting of leg cramps, nausea, headache, and dizziness associated with hPTH administration.
[15] A pharmaceutical composition for transmucosal administration containing hPTH or a derivative thereof, wherein the composition is administered for purposes of maintaining an action of hPTH for bone mass increase or bone density increase, and reducing a probability of developing one or more symptoms selected from the group consisting of leg cramps, nausea, headache, and dizziness associated with hPTH administration.
[16] A pharmaceutical composition for transmucosal administration containing hPTH or a derivative thereof, wherein the composition is administered for purposes of maintaining an action of hPTH for bone mass increase or bone density increase, and reducing a frequency of developing one or more symptoms selected from the group consisting of leg cramps, nausea, headache, and dizziness associated with hPTH administration.
[17] A pharmaceutical composition for transmucosal administration containing hPTH· or a derivative thereof, wherein the composition is administered for a purpose of maintaining both actions of hPTH for bone formation and bone resorption inhibition.
[18] The pharmaceutical composition of any of [11] to [17], wherein transmucosal administration is by a nasal mucosa.
[19] The pharmaceutical composition of any of [11] to [18], wherein hPTH is hPTH1-34.
[20] The pharmaceutical composition of any of [11] to [19], wherein a dose of the composition per day is 250 µg to 1,000 µg.
[21] The pharmaceutical composition of any of [11] to [19], wherein the composition is formulated such that a dose of the composition per day is 250 µg to 1,000 µg.
[22] A method for reducing a risk of developing one or more symptoms selected from the group consisting of leg cramps, nausea, headache, and dizziness associated with hPTH administration, the method comprising administering hPTH or a derivative thereof by transmucosal administration.
[23] A method for reducing a probability of developing one or more symptoms selected from the group consisting of leg cramps, nausea, headache, and dizziness associated with hPTH administration, the method comprising administering hPTH or a derivative thereof by transmucosal administration.
[24] A method for reducing a frequency of developing one or more symptoms selected from the group consisting of leg cramps, nausea, headache, and dizziness associated with hPTH administration, the method comprising administering hPTH or a derivative thereof by transmucosal administration.
[25] The method according to any of [22] to [24], wherein an action of hPTH for bone mass increase or bone density increase is maintained.
[26] A method for maintaining both functions for bone formation and bone resorption inhibition, comprising administering hPTH or a derivative thereof by transmucosal administration.
[27] The method of any of [22] to [26], wherein transmucosal administration is by a nasal mucosa.
[28] A method for maintaining an inhibition function for bone resorption, comprising administering hPTH or a derivative thereof by an administration method having a shorter half life in blood in comparison with subcutaneous administration.
[29] A method for maintaining bone formation function and bone resorption inhibition function, comprising administering hPTH or a derivative thereof by an administration method having a shorter half life in blood in comparison with subcutaneous administration.
[30] The method of any of [22] to [29], wherein hPTH is hPTH1-34.
[31] The method of any of [22] to [30], wherein a dose of the composition per day is 250 µg to 1,000 µg.

As shown in Example 1, transmucosal administration of a pharmaceutical composition of the invention containing hPTH significantly reduces a probability and a frequency of developing a symptom such as nausea, leg cramps, headache, and dizziness, which are developed when being administered through administration routes other than transmucosal administration. Also, target actions of hPTH such as bone mass increase action, bone density increase action, and bone resorption inhibiting action can be maintained.

Further, as shown in Examples 2 to 4, an administration method allowing a drug to have a short half life in blood can maintain both bone formation action and bone resorption inhibiting action of hPTH. Examples of administration methods providing a short half life in blood include transmucosal administration and intravascular administration.

That is, when the pharmaceutical composition of the invention containing hPTH is administered by an administration method providing a short half life such as transmucosal administration, both bone formation action and bone resorption inhibiting action of hPTH can be maintained.

This description includes the contents disclosed in the description and/or drawings of Japanese Patent Application No. 2004-207733, which is a priority document of the present application.

### Brief Description of the Drawings

Figure 1 is a view showing changes of bone density at 12 weeks after administration of a pharmaceutical composition of the invention;
Figure 2A is a view showing changes of blood PINP as bone formation marker at 6 weeks after administration;
Figure 2B is a view showing changes of blood PINP as bone formation marker at 12 weeks after administration;
Figure 3A is a view showing changes of urine NTx as bone resorption marker at 6 weeks after administration;
Figure 3B is a view showing changes of urine NTx as bone resorption marker at 12 weeks after administration;
Figure 4 is a view showing lumbar vertebra bone density increase action of hPTH(1-34) in aged OVX rats;
Figure 5 is a view showing effects of hPTH(1-34) on bone resorption marker in aged OVX rats;
Figure 6 is a view showing effects of hPTH(1-34) on bone formation marker in aged OVX rats;
Figure 7A is a view showing the time course of hPTH(1-34) levels in blood through iv administration in rats;
Figure 7B is a view showing the time course of hPTH(1-34) levels in blood through sc administration in rats;
Figure 8 is a view showing lumbar vertebra bone density increase action of hPTH(1-34) in aged OVX rats;
Figure 9A is a view showing effects of hPTH(1-34) on a bone resorption marker (DPD/Cre) in aged OVX rats;
Figure 9B is a view showing effects of hPTH(1-34) on a bone resorption marker (TRACP5b) in aged OVX rats;
Figure 10 is a view showing effects of hPTH(1-34) on a bone formation marker (osteocalcin) in aged OVX rats;
Figure 11A is a view showing effects of PTH(1-34) on a bone mass parameter (BV/TV) in aged OVX rats;
Figure 11B is a view showing effects of PTH(1-34) on a bone mass parameter (Tb.Th) in aged OVX rats;
Figure 12A is a view showing effects of PTH(1-34) on a bone resorption parameter (ES/BS) in aged OVX rats;
Figure 12B is a view showing effects of PTH(1-34) on a bone resorption parameter (N.Oc/BS) in aged OVX rats;
Figure 13A is a view showing effects of PTH(1-34) on a bone formation parameter (BFR/BS) in aged OVX rats; and
Figure 13B is a view showing effects of PTH(1-34) on a bone formation parameter (LS/BS) in aged OVX rats.

### Best Mode for Carrying Out the Invention

Hereinafter, the present invention will be described in detail.

As described above, administration of hPTH reportedly increases a risk of developing symptoms such as nausea, leg cramps, headache, dizziness, etc., and increases a probability of developing the symptoms, or a frequency of developing the symptoms, in comparison with no administration of hPTH.

A pharmaceutical composition of the invention for transmucosal administration containing hPTH as an active ingredient can reduce a risk of developing symptoms such as nausea, leg cramps, headache, dizziness, etc., reduce a probability of developing the symptoms, and reduce a frequency of developing the symptoms, while maintaining an action of hPTH.

The maintenance of action is not restricted by the degree of such action, as long as such action is observed. Preferably, the degree of the action is maintained such that therapeutic effectiveness is recognized (for example, therapeutic effectiveness on osteoporosis).

In the invention, the expression "action of hPTH" means bone mass increase, bone density increase, bone resorption inhibition or the like. When hPTH maintains these actions, it can be used for the purpose of treating osteoporosis or avoiding fracture. In the invention, to maintain an action of hPTH means that when hPTH is administered, the action of hPTH is observed in a hPTH-administered patient. Bone mass increase action, bone density increase action, and bone resorption inhibiting action can be measured by methods known to a skilled person, and, for example, by methods described in Examples.

In the invention, the expression "to inhibit an increase of a risk of developing symptoms" means to inhibit an increase of a risk of developing unintended symptoms with hPTH administration. In the invention, the expression "to inhibit an increase" means to eliminate an increase itself or to reduce the degree of increase. The expression "risk of developing symptoms" means a risk which may cause unintended symptoms with hPTH administration. In the invention, regarding as standard a case wherein hPTH is administered by administration routes (such as subcutaneous administration) other than transmucosal administration, a reduced risk of developing unintended symptoms is considered "a risk of developing symptoms is reduced".

According to the invention, it has been found that transmucosal administration can reduce a risk of developing unintended symptoms which may be increased when hPTH is administered by administration routes (such as subcutaneous administration) other than transmucosal administration.

In the invention, the expression "to reduce a frequency of developing symptoms" means to reduce a proportion of patients developing unintended symptoms in a patient group with hPTH administered. Therefore, the frequency of developing symptoms indicates a proportion of patients who develop symptoms among the patient group with hPTH administered. For example, when, among 100 patients with hPTH administered, a certain symptom is observed in 10 patients, the frequency of developing that symptom is 10%. In this case, "to reduce a frequency of developing the symptom" means to reduce a frequency to less than 10% (that is, the symptom is observed in 9 patients or less).

Whether the frequency of developing a symptom is reduced or not is determined based on the frequency of developing the symptom when hPTH is administered by administration routes (such as subcutaneous administration) other than transmucosal administration (particularly transnasal administration).

More specifically, it has been reported that subcutaneous administration of hPTH1-34 causes headache, nausea, dizziness, and leg cramps at frequencies of 13%, 18%, 9%, and 3%, respectively (Robert M Neer et al., N Engl J Med, 344(19), 1434-1441, (2001)). Therefore, in a preferred embodiment to "reduce a frequency of developing a symptom" in the invention: the frequency of headache is reduced to less than 13%, preferably less than 10%, and more preferably less than 9%; the frequency of nausea is reduced to less than 18%, preferably less than 10%, and more preferably less than 5%; the frequency of dizziness is reduced to less than 9%, preferably less than 7%, and more preferably less than 6%; and the frequency of leg cramps is reduced to less than 3%, preferably less than 2%, and more preferably less than 1%.

For example, in the case of transnasal administration, the frequencies for developing these symptoms are 7.2% for headache, 0% for nausea, 4.1% for dizziness, and 0% for leg cramps, which are greatly reduced in comparison with cases with subcutaneous administration, thereby enabling the reduction of a risk of developing the symptoms.

Further, the expression "to reduce a probability of developing a symptom" in the invention means to reduce a probability of developing an unintended symptom in a certain patient. Therefore, in the case of "reducing a frequency of developing a symptom", a group (patient group) is a subject. On the other hand, in the case of "reducing a probability of developing a symptom", a certain individual patient is a subject. Thus, "the probability of developing a symptom" represents a proportion at which a certain patient develops a symptom when hPTH is administered to the patient. Usually, whether "the probability of developing a symptom is reduced" or not can be determined from a proportion of patients developing the symptom in the patient group with hPTH administered in the same manner as the case for "reducing a frequency of developing a symptom". Further, whether the probability of developing a symptom is reduced or not is determined based on a proportion at which a certain symptom is developed in a group with hPTH administered by an administration route (for example, subcutaneous administration) other than transmucosal administration (particularly transnasal administration). In the invention, the number of a group (patient group) is not limited, but it is usually 5 or more patients, preferably 15 or more patients, more preferably 20 or more patients, and still more preferably 25 or more patients.

As described above, transmucosal administration of the pharmaceutical composition of the invention reduces a probability or a frequency of developing a symptom in comparison with cases wherein the composition is administered by administration routes other than transmucosal administration. For example, one or more symptoms selected from the group of leg cramps, nausea, headache, and dizziness is eliminated, or the probability and the frequency of developing the symptoms are reduced close to a half.

In the invention, an unintended symptom, which is a target for reducing the probability and the frequency of its onset, is an undesirable symptom that is developed at a high frequency by administering hPTH through administration routes (for example, subcutaneous administration) other than transmucosal administration. Examples thereof include leg cramps, nausea (bout of vomiting, vomiting, gastric distress, etc.) headache, and dizziness.

Further, transmucosal administration, such as transnasal administration of hPTH can reduce onset of the above-described undesirable symptoms while maintaining bone mass increase action, bone density increase action, or bone resorption inhibiting action of hPTH.

Furthermore, usually, when administration of hPTH inhibits bone resorption, the inhibition of bone formation also occurs. Thus, it was impossible to maintain both bone resorption inhibiting action and bone formation action of hPTH.

However, in the invention, it has been found that hPTH is administered by an administration method enabling a short half life in blood, and thereby both bone formation action and bone resorption inhibiting action of hPTH can be maintained.

In the invention, an administration method having a short half life in blood is an administration method enabling a shorter half life in blood compared with subcutaneous injection. Specific examples of such methods include transmucosal administration (such as transnasal administration) and intravascular administration (such as intravenous administration).

A half life in blood in hPTH administration can be measured by methods known to a skilled person, and, for example, a half life in blood can be measured by the method described in Examples.

Therefore, the invention relates to a method for maintaining bone resorption inhibiting action by administering hPTH or its derivative through an administration method enabling a short half life in blood, and in particular, to a method for maintaining both bone resorption inhibiting action and bone formation action.

A preferred embodiment of the invention includes a method for both bone formation action and bone resorption inhibiting action by transmucosally administering hPTH or its derivative.

hPTH to be used in the invention can be any hPTH, and examples thereof include a full-length hPTH, hPTH derivatives, modified hPTHs. Further, the examples include naturally-occurring PTH, recombinant PTHs produced by genetic engineering techniques, and chemically synthetic PTHs.

Examples of hPTH derivatives include hPTH(1-84) (Biochemistry 17, 5723(1978), Kimura et al.; Biochem, Biophys. Res. Commun., vol. 114, p. 493, 1983), hPTH(1-38) (JP Patent Publication (Kokai) No. 57-81448 A (1982), hPTH(1-34) (JP Patent Publication (Kokai) No. 9-29600 A (1997); Takai et al., Peptide Chemistry, 1979, p187), hPTH(1-34)NH₂ (JP Patent Publication (Kokai) No. 58-96052 A (1983)), [Nle^{8,18}]hPTH(1-34), [Nle^{8,18},Tyr³⁴]hPTH(1-34) (JP Patent Publication (Kokai) No. 55-113753 A (1980)), [Nle^{8,18}]hPTH(1-34)NH₂ (JP Patent Publication (Kokai) No. 61-24598 A (1986)), [Nle^{8,18},Tyr³⁴]hPTH(1-34)NH₂ (JP Patent Publication (Kokai) No. 60-34996 A (1985), hPTH(1-37) (JP Patent Publication (Kohyo) No. 5-505594 A (1993)), hPTH(2-84), hPTH(3-84), hPTH(4-84), hPTH(5-84), hPTH(6-84), hPTH(7-84), and hPTH(8-84) (JP Patent Publication (Kohyo) No. 4-505259 A (1992)). Further, examples of the inventive hPTH include those formed by substitution of some constituent amino acids with other amino acids, deletion of some constituent amino acids, and addition of at least one amino acid to the constituent amino acid in the above hPTHs and having comparable activities. Preferred examples of amino acid substitution is substitution of the constituent amino acid at 8-position with leucine or norleucine, substitution of the constituent amino acid at 18-position with leucine or norleucine, and substitution of the constituent amino acid at 34-position with tyrosine.

A preferred hPTH for the invention is hPTH(1-34).

The hPTHs can be produced by methods known to a person skilled in the art. For example, these can be produced by methods based on genetic engineering techniques or chemical synthesis techniques (JP Patent Publication (Kokai) No. 9-296000 A (1997), JP Patent No. 2643968, etc.). The produced hPTH may be purified using a known technique such as column chromatography. The hPTH is a basic peptide, so an acid such as acetic acid may be used as an eluent to prevent adsorption of hPTH to a column resin. When an acid is used at the time of purification, it is desirable to reduce an amount of the acid in a pharmaceutical composition containing hPTH. Reduction of the acid can be achieved by known methods such as dialysis, electrodialysis, ion exchange chromatography, gel filtration, and reverse phase HPLC.

The pharmaceutical composition of this invention for transmucosal administration containing hPTH as an active ingredient, has a good tolerance for change of the formulation, and thus it can properly be mixed with a component commonly used for formulation, such as a carrier, an excipient, a thickener, a preserver, a stabilizer, an antioxidant, a binder, a disintegrant, a humectant, a lubricant, a colorant, a flavoring agent, a corrigent, a suspending agent, an emulsifying agent, a solubilizer, a buffering agent, a tonicity agent, a surfactant, a soothing agent, and a sulfur-containing reducing agent. Further, the pharmaceutical composition can properly mixed with various functional components for the purpose of absorption improvement, solid stability, or the like.

Examples of the carrier or excipient include substances well or sparingly soluble in water, such as sugars, polysaccharides, dextrins, celluloses, synthetic or semisynthetic polymers, amino acids, polyamino acids, proteins, and phospholipids.

Examples of the sugars (monosaccharides, oligosaccharides) include D-mannitol, glucose, lactose, fructose, inositol, sucrose, maltose, while the examples of polysaccharides include dextran, pullulan, alginic acid, hyaluronic acid, pectic acid, phytic acid, and phytin. Examples of the dextrins include α-cyclodextrin, β-cyclodextrin, γ-cyclodextrin, dextrin, hydroxypropylstarch, and hydroxyethylstarch.

Examples of the celluloses include methylcellulose, ethylcellulose, hydroxyethylcellulose, hydroxypropylcellulose, hydroxypropylmethylcellulose, and sodium carboxymethylcellulose.

Examples of the synthetic or semisynthetic polymers include polyvinyl alcohol, carboxyvinyl polymer, polyethylene glycol, polyvinyl pyrrolidone (PVP), sodium polyacrylate, and polylactic acid.

Examples of the amino acids include glycine and taurine, while examples of the polyamino acids include polyglutamic acid, polyaspartic acid, polyglycine, and polyleucine.

Examples of the proteins include gelatin and others. In addition, chitin and chitosan may be included.

Of these carriers or excipients, particularly preferred are sucrose, maltose, α-cyclodextrin, β-cyclodestrin, dextrin, D-mannitol, inositol, lactose, dextran, methylcellulose, hydroxypropylcellulose, polyvinyl alcohol, and pullulan.

Besides them, usable are sorbic acid; benzalconium chloride; cetylpyridinium chloride; benzethonium chloride; parabens such as methyl para-hydroxybenzoate, ethyl para-hydroxybezoate, propyl para-hydroxybenzoate, butyl para-hydroxybenzoate, isobutyl para-hydroxybenzoate and others; gum acacia; sorbitol; magnesium stearate; talc; silica; microcrystalline cellulose; starch; calcium phosphate; vegetable oil; carboxymethylcellulose; sodium lauryl sulfate; water; ethanol; glycerin; and syrup.

Typical examples of surfactants are listed below. Among these, single or combination of two or more of these surfactants can be added to the formulation in the invention. Examples of nonionic surfactants include: sorbitan esters of fatty acids such as sorbitan monocaprilate, sorbitan monolaurate, and sorbitan monopalmitate; glycerol esters of fatty acids, such as glyceryl monocaprilate, glyceryl monomyristate, and glyceryl monostearate; polyglycerol esters of fatty acids, such as decaglyceryl monostearate, decaglyceryl distearate, and decaglyceryl monolinoleate; polyoxyethylene sorbitan esters of fatty acids, such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, and polyoxyethylene sorbitan tristearate; polyoxyethylene sobitol esters of fatty acids, such as polyoxyethylene sobitol tetrastearate and polyoxyethylene sobitol tetraoleate; polyoxyethylene glycerol esters of fatty acids, such as polyoxyethylene glyceryl monostearate; polyethylene glycerol esters of fatty acids, such as polyethylene glycol distearate; polyoxyethylene alkyl ether, such as polyoxyethylene lauyl ether; polyoxyethylene polyoxypropylene alkyl ether, such as polyoxyethylene polyoxypropylene glycol ether, polyoxyethylene polyoxypropylene propylether, and polyoxyethylene polyoxypropylene cetyl ether; polyoxyethylene alkylphenyl ether, such as polyoxyethylene nonylphenyl ether; polyoxyethylene hardened caster oils, such as polyoxyethylene caster oil and polyoxyethylene hardened caster oil (polyoxyethylene hydrogenated caster oil); polyoxyethylene beeswax derivatives, such as polyoxyethylene sorbitol beeswax; polyoxyethylene lanolin derivatives, such as polyoxyethylene lanolin; and polyoxyethylene amides of fatty acids with HLB 6 to 18 such as polyoxyethylene stearylamide. Examples of anionic surfactants include alkyl sulfate salts having an alkyl group containing 10 to 18 carbon atoms, such as sodium cetyl sulfate, sodium lauryl sulfate, and sodium oleylsulfate; and polyoxyethylene alkylether sulfate salts having 2 to 4 moles of ethyleneoxide added on average and an alkyl group containing 10 to 18 carbon atoms, such as sodium polyoxyethylene lauryl sulfate; and alkyl sulfo succinate ester salts having an alkyl group containing 8 to 18 carbon atoms, such as sodium lauryl sulfo succinic acid ester. Examples of naturally occurring sulfactants include lecithin; glycerophospholipids; sphingolipids, such as sphingomyelin; and sucrose esters of fatty acids having a fatty acid containing 12 to 18 carbon atoms. Examples of sulfur-containing reducing agents include N-acety cysteine, N-acety homocysteine, thioctic acid, thiodiglycol, thioethanolamine, thioglycerol, thiosorbitol, thioglycolic acid and its salts, sodium thiosulfate, glutathione, thioalkanic acids containing 1 to 7 carbon atoms having a sulfhydryl group.

Examples of antioxidants include erysorbic acid, dibutylhydroxytoluene, butylhydroxyanisole, α-tocopherol, tocopherol acetate, L-ascorbic acid and its salts, L-ascorbyl palmitate, L-ascorbyl stearate, sodium bisulfite, sodium sulfite, triamyl gallate, propyl gallate, and chelating agents, such as disodium ethylenediamine tetraacetate (EDTA), sodium pyrophospate, and sodium metaphosphate.

With respect to the ratio of each component in the pharmaceutical composition of this invention, hPTH may be present at about 0.01 to 20%, preferably at about 0.05 to 10%. An organic acid may be added as appropriate, and, when it is added, it may be present at about 0.05 to 99.5%, preferably about 0.1 99.0%. A carrier or excipient which is usually used in preparation of a medicinal product may be added as appropriate, and, when it is added, it may be present, for example, at about 0.01 to 99.5%. Other various functional components may be added as appropriate, and, in the case of their addition, they may be present, for example, at about 0.05 to 99.5%.

The pharmaceutical composition of the invention containing hPTH as an active ingredient, may be administered through transmucosal administration. Any administration method can be used, such as transnasal administration, transpulmonary administration, transrectal administration, sublingual administration, and buccal administration, as long as the pharmaceutical composition is administered through a mucosa.

In the present invention, a preferred transmucosal administration is transnasal administration.

The formulation for transnasal administration is not particularly limited, and examples thereof include a droplet, a spray, a mist, a coating, a powder and a gel. The composition is absorbed through a tissue and/or a vascular in the nose and/or sinus tract.

The pharmaceutical composition of hPTH for transnasal administration can be produced by a known method (see WO02/02136, etc.).

The pharmaceutical composition of the invention for transnasal administration can be prepared in accordance with a known method.

For example, an hPTH pharamceutical component with a lower acetic acid content may be served as a pharmaceutical composition as it is. Alternatively, a carrier or excipient commonly used for formulation, and an organic acid and other various functional components, may be added to and mixed with, as appropriate, the hPTH pharmaceutical component with a lower acetic acid content, and the resulting product may be used as a pharmaceutical component. The mixing is carried out by displacement of the organic acid with acetic acid or simply by addition. For example, a mixture containing a carrier or excipient commonly used for formulation, an organic acid, and various functional components if they are necessary, together with the hPTH pharmaceutical component is first dissolved in distilled water. The solution is then lyophilized to obtain a uniform composition.

Alternatively, an hPTH pharmaceutical component, and a carrier or excipient commonly used for formulation if necessary are first dissolved in distilled water and then lyophilized. Thereafter, an organic acid and various functional components may be optionally added to the lyophilizate and dissolved together, and then lyophilized to obtain a uniform composition.

As a further alternative, an hPTH pharmaceutical component, and an organic acid or various functional components are first dissolved in distilled water and then lyophilized. Then, a carrier or excipient commonly used for formulation if necessary is dissolved with the obtained product, and lyophilized, thereby obtaining a uniform composition.

The pharmaceutical component of the invention can be formulated in various dosage forms depending on the type of administration method, and it can be formulated in dosage forms capable of transmucosal administration through rectum, nasal cavity, and oral cavity. Further, the pharmaceutical composition of the invention for transnasal administration is preferably administered in the form of a transnasal drug.

A preferred example of the pharmaceutical composition of the invention for transnasal administration is a formulation dissolved before use, which contains the pharmaceutical component of the invention provided as a lyophilized composition in a lyophilized portion and has a dissolving solution portion attached thereto.

The above-mentioned organic acid and the absorption-promoting organic acid such as citric acid, adipic acid and glycolic acid may be a part of the pharmaceutical component of the invention as a salt of hPTH, an attachment, or an additive in the lyophilized portion. Alternatively, these may be added to and dissolved in the dissolving solution portion.

Further, the pharmaceutical composition of the invention for transnasal administration may be administered by a known method. For example, the pharmaceutical composition of the invention for transnasal administration may be contained in a formulation used as a transnasal drug. For example, intranasal administration method may be used by spraying the composition. A container having the pharmaceutical composition is provided with a nebulizer, and a tip of a nozzle is inserted into a nasal cavity for spraying.

A dose of the pharmaceutical composition of the invention may vary depending on the kind of disease, the age and weight of a patient, the severity of disease, and the administration route. For example, when hPTH(1-34) is transnasally administered, it may be administered once or several times a day on consecutive days. The administration is preferably carried out such that a single dose contains hPTH(1-34) at 10 µg to 5,000 µg, preferably 250 µg to 1,000µg. Further, after a certain period of withdrawal, administration may be resumed depending on the symptom.

Further, a dose per day is not particularly limited, and can be determined properly by a person skilled in the art. The administration is carried out such that an amount per day of hPTH or its derivatives is 250 µg to 1,000 µg. For such administration, a pharmaceutical formulation is prepared such that a single dose contains, for example, 250 µg to 1,000 µg of the pharmaceutical composition of the invention, and then administered once per day.

### EXAMPLES

The present invention will be described in detail by referring to examples, but the invention is not limited thereto.

### Example 1 Effect on patient with primary osteoporosis

Effects of the pharmaceutical composition of the invention were examined in accordance with the following method.
Subject: patients with primary osteoporosis
Design: Comparison test among randomly allotted parallel groups
Usage and Dose: daily transnasal administration of 250 µg, 500 µg, or 1,000 µg of hPTH(1-34)

Specifically, a lyophilized composition containing hPTH(1-34) was prepared so as to contain 250 µg, 500 µg, or 1,000 µg of hPTH(1-34) in 200 µl of drug solution when dissolved in a dissolving solution, and the prepared composition was dissolved before use and administered. Here, hPTH1-34 transnasal administration formulation was produced by the method in Example 2 of International Patent Publication No. WO02/02136. Using a nebulizer VP-7 (Valois) which uniformly sprays 100 µL of the drug solution by pumping once, one pumping for each nasal cavity, the total of 200 µL of the drug solution was sprayed daily.

Examination method: patients with primary osteoporosis were randomly allotted to three groups: 250-µg group, 500-µg group; and 1,000-µg group. A single dose containing each amount of hPTH(1-34) was administered once a day on consecutive days of 12 weeks, and effectiveness and safety of each amount was confirmed.

Evaluation of effectiveness: the change rate of bone mineral density (BMD), the change rate of blood PINP as bone formation marker, and the change rate of blood NTx as bone resorption marker were evaluated for 12 weeks.

Specifically, the change rate of the second through fourth lumber vertebral bom mineral density (L2-4BMD) after 12-week administration measured by DXA method, the change rate of blood PINP as bone formation marker measured by RIA method, and the change rate of urine NTx as bone resorption marker measured by ELISA method were evaluated. For PINP and NTx measurement, UniQ PINP RIA (available from Orion Diagnostica) and Osteomark (available from Mochida Pharmaceutical Co., Ltd.) were used, respectively.

Safety evaluation: the occurrence number of adverse events was counted and evaluated.

The following results were obtained.

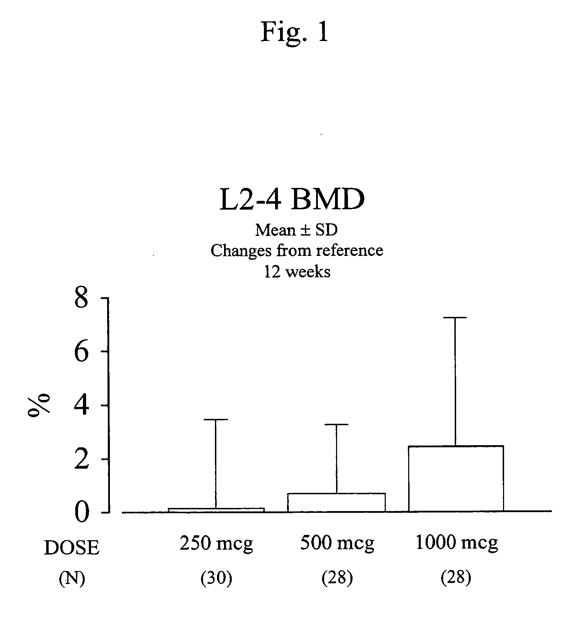

Regarding the change rate (average) of BMD after 12-week administration, the 250-, 500-, and 1,000-µg groups indicated 0.14%, 0.69%, and 2.44%, respectively, and increased in a dose-dependant manner. The change rate of the 1,000-µg group significantly increased compared with the beginning of administration. Further, the 1,000-µg group showed a significantly high increase rate compared with the 250-µg group. Figure 1 shows the change rates of BMD.

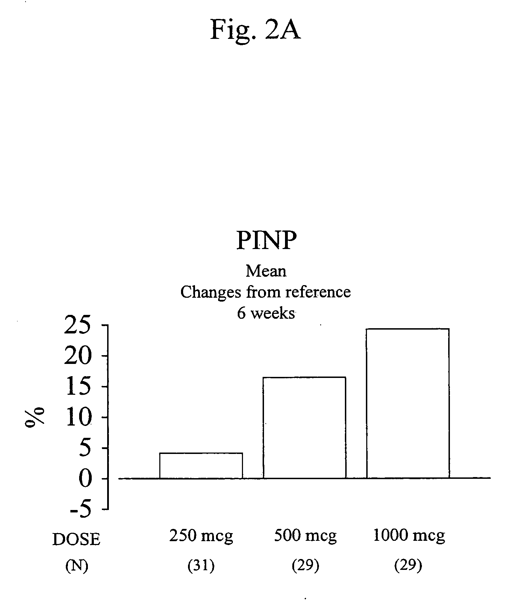

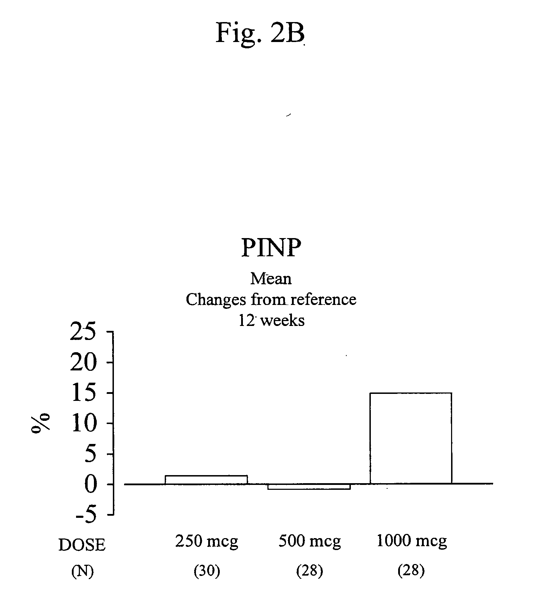

Regarding the change rate (median) of blood PINP as blood formation marker at the time of 6-week administration, the 250-, 500-, and 1,000-µg groups indicated 4.1%, 16.5%, and 24.3%, respectively. Significant increases compared with before administration were observed in the 500 µg group and the 1,000-µg group. The change rates at the time of 12-week administration were 1.4%, -0.84%, and 14.8% for the 250-, 500-, and 1,000-µg groups, respectively. A significant increase compared with before administration was observed in the 1,000-µg group. Figure 2 shows the change rates of blood PINP.

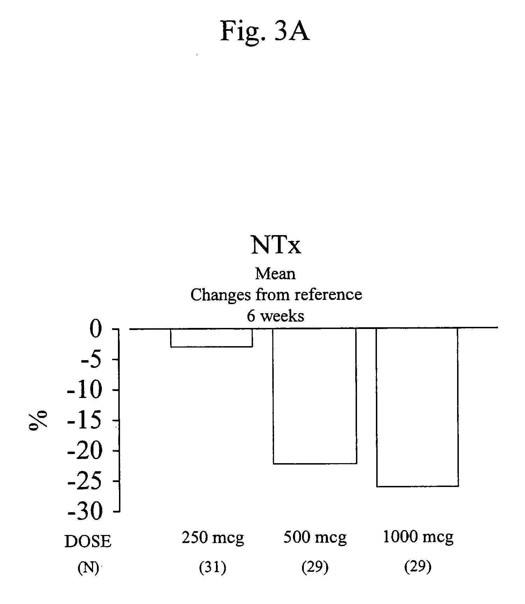

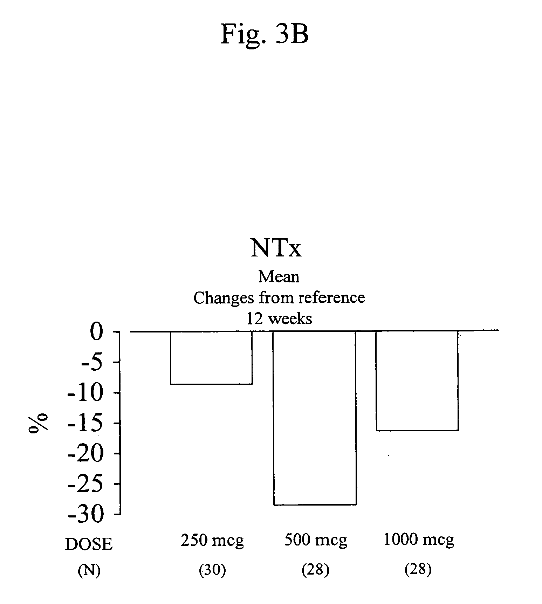

Regarding the change rate (median) of urine NTx as bone resorption marker at the time of 6-week administration, the 250-, 500-, and 1,000-µg groups indicated -3.0%, -22.2%, and -26.1%, respectively, and the 500-µg group had a significantly large decreasing rate compared with the 250-µg group. Significant decreases compared with before administration were observed in the 500-µg group and the 1,000-µg group. The change rates at the time of 12-week administration were -8.7%, -28.6%, and -16.4% for the 250-, 500-, and 1,000-µg groups, respectively. The 500-µg group had a significantly large decreasing rate compared with the 250-µg group. Significant decreases compared with before administration were observed in the 500-µg group and the 1,000-µg group. Figure 3 shows the change rates of urine NTx.

In counting adverse and other events, coding to preferred terms (PT) and classification of adverse event names into system organ classes (SOC) were conducted based on "MedDRA Version6.1".

Among 97 subjects for safety evaluation, 155 adverse events occurred in 70 subjects (72.2%). The occurrence numbers of respective dose groups are as follows: the 250-µg group had 46 events in 22 subjects (71.0%) from 31 subjects; the 500-µg group had 51 events in 23 subjects (76.7%) from 30 subjects; the 1,000-µg group had 48 events in 20 subjects (69.0%) from 20 subjects; and 1,500-µg group had 10 events in 5 subjects (71.4%) from 7 subjects. Among these, the events (PT indication) with the occurrence rate of 5% or more included 14 events (14.4%) of nasopharyngitis, 7 events (7.2%) of headache, 12 events (12.4%) of supraventricular premature beat, and 5 events (5.2%) of ventricular premature beat. According to the counting by SOC, 21 events (21.6%) of heat failure, 19 events (19.6%) of clinical examination, and 18 events (18.6%) of infectious disease and parasitic disease were observed.

It has been reported that leg cramps (3%), nausea (18%), dizziness (9%), headache (13%) and the like caused by subcutaneous administration were observed (Reference document: Robert M Neer et al., N Engl J Med, 344(19), 1434-1441, (2001). However, change of administration routs to transnasal administration resulted in no observation of leg cramps and nausea, and remarkable decreases in occurrence rates of dizziness (4.1%) and headache (7.2%).

Regarding side effects, 37 events occurred in 24 subjects (24.7%). The occurrence numbers of respective dose groups are as follows: the 250-µg group had 12 events in 7 subjects (22.6%) from 31 subjects; the 500-µg group had 10 events in 8 subjects (26.7%) from 30 subjects; the 1,000-µg group had 13 events in 7 subjects (24.1%) from 29 subjects; and 1,500-µg group had 2 events in 2 subjects (28.6%) from 7 subjects. Among these, no event with the occurrence rate of 5% or more was observed.

According to the results of effectiveness and safety in the clinical examination, it has been concluded that the agent can remarkably increase BMD by facilitating bone formation and suppressing bone resorption and is excellent in safety.

### Example 2 Effect of caudal vein administration of PTH(1-34) on bone metabolism in aged OVX rats

PTH(1-34) was administered to aged OVX rats by caudal vein administration (iv), and effects of PTH(1-34) on bone metabolism were examined.

Female SD-IGS rats at 34 weeks of age (Charles River Japan, Inc.) were ovariectomized (OVX) to remove both ovaries or sham-operated. At 48 weeks of age, the OVX group was measured for bone density and subdivided into groups of 8 rats each such that the mean BMD of each group was identical.

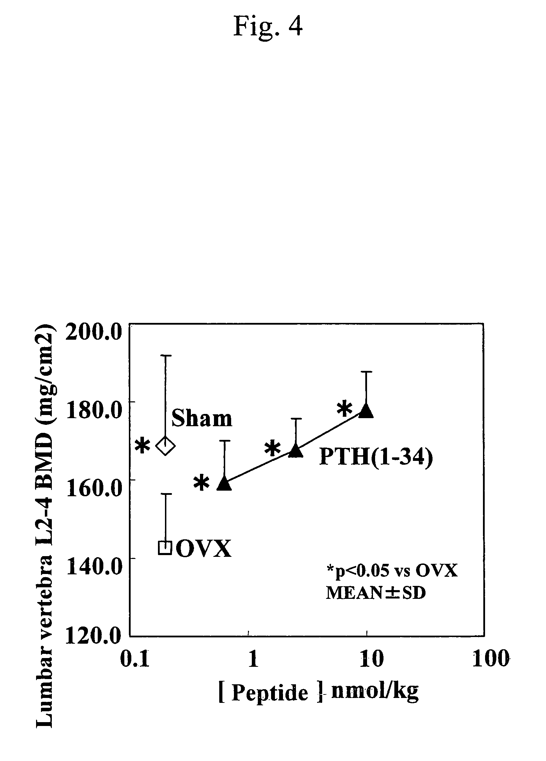

hPTH(1-34) was diluted with phosphate buffer solution (PBS)/0.05% Tween 80 and adjusted to 10, 2.5 0.625 nmol/ml. Phosphate buffer solution (PBS)/0.05% Tween 80 was administered to the 8 rats of each of the sham group and the OVX group. The diluted hPTH(1-34) was administered by caudal vein administration to groups of 8 rats each at a dose of 1 ml/kg (10, 2.5, 0.625 nmol/kg) on a 5 times-a-week basis for 6 weeks. On the last day of administration, the rats were housed in metabolic cages and 24-hour urine was collected from each rat. On the following day, after the rats were euthanized by exsanguination under anesthesia, an autopsy was performed to collect blood, lumbar vertebrae and femurs. The urine and blood were taken into tubes and centrifuged to collect the respective supernatants, which were stored at -20°C until assayed for parameters. The lumbar vertebrae and right femurs were stored in 70% ethanol. The average bone density of the second to fifth lumbar vertebrae and the bone density of the right femur were measured using a dual X-ray bone mineral densitometer (DCS-600EX, ALOKA). The bone metabolism marker in serum and urine was measured. Figure 4 shows the results thereof.

As shown in Figure 4, the OVX group showed a significant decrease in lumbar bone density over the sham group. Further, administration of hPTH(1-34) caused a significant, dose-dependent increase in lumbar bone density of the OVX group.

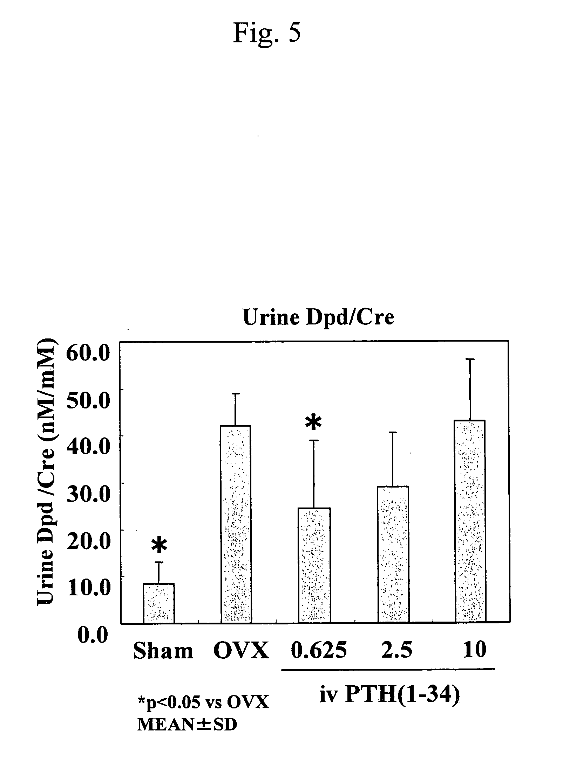

Next, deoxypyridinoline (DPD) in urine as bone resorption marker was measured. Figure 5 shows the results thereof. DPD was corrected by urine creatinine value. Regarding urine DPD, a significant decrease was observed in the iv administration group of 0.625 nmol/kg PTH(1-34). However, no significant decreases were observed at doses of 2.5 nmol/kg and 10 nmol/kg.

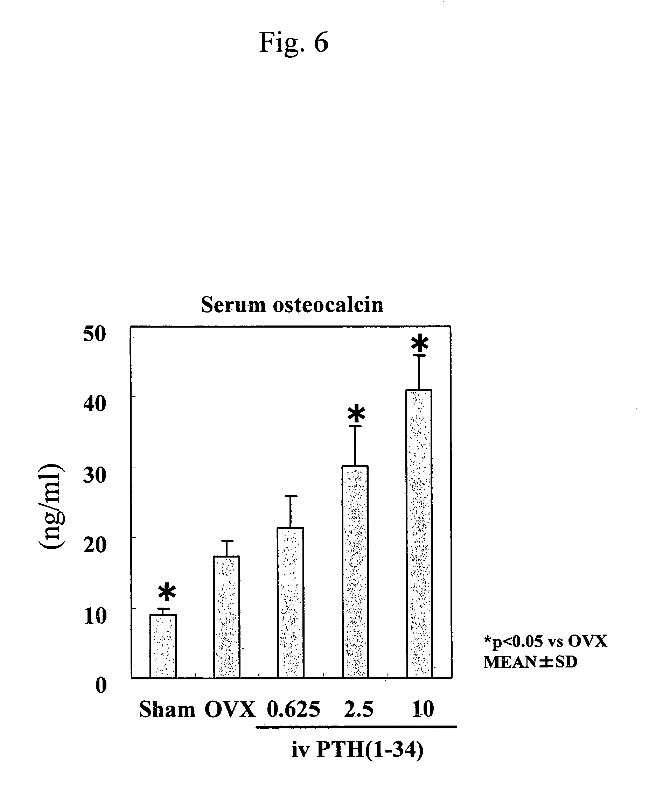

Then, osteocalcin (OC) in blood as bone formation marker was measured. Figure 6 shows the results thereof. The OVX group showed significant increases over the sham group. Further, significant increases were observed in the PTH(1-34) administration groups at doses of 2.5 nmol/kg and 10 nmol/kg over the OVX group.

As described above, iv administration of PTH(1-34) increased BMD, while bone formation marker increased and bone resorption marker decreased. This has suggested that iv administration of PTH(1-34) inhibits bone resorption at a low dose.

### Example 3 Changes of PTH(1-34) level in plasma by caudal vein and subcutaneous administration

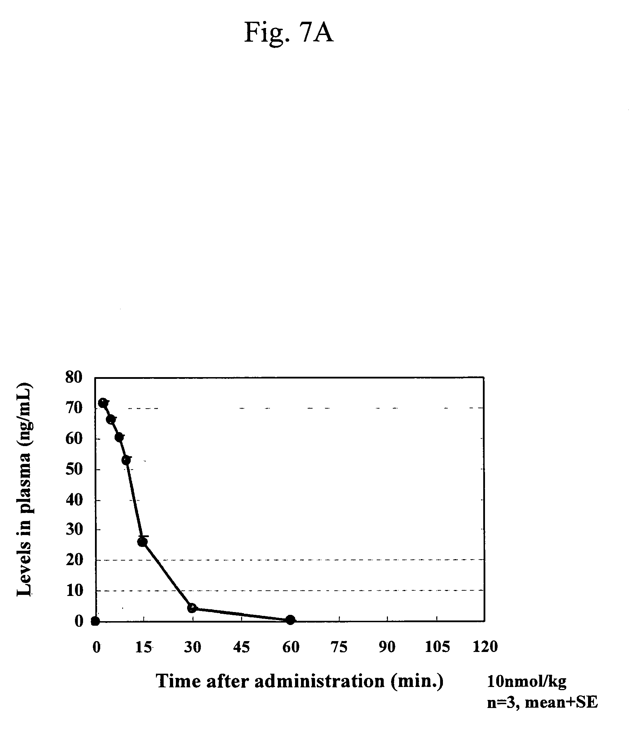

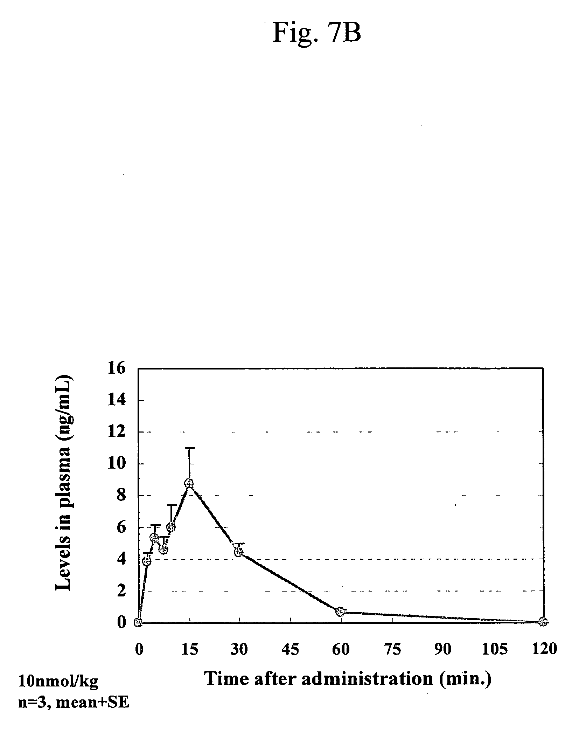

Changes of PTH(1-34) level in plasma by caudal vein administration (iv) and subcutaneous administration (sc) were examined. hPTH(1-34) was diluted with phosphate buffer solution (PBS)/0.05% Tween 80 and adjusted to 10 nmol/ml. Female SD-IGS rats at 8 weeks of age (Charles River Japan, Inc.) were used for the experiment. A single dose administration was carried out by caudal vein administration and subcutaneous administration, and blood was collected from tail vein using a capillary tube for blood collection in a time-dependent manner (at pre-administration, 2.5, 5, 7.5, 10, 15, 30, 60, 90, 120 min.). After EDTA plasma was isolated, the collected blood samples were stored at -80°C until the hPTH(1-34) level was measured. The PTH(1-34) level was measured by ELISA method using PTH(1-34) (human)-EIA kit (Peninsula Laboratories). Changes of PTH(1-34) level in plasma and pharmacokinetics parameters calculated therefrom are shown in Figures 7A and 7B, and Table 1.

It was confirmed that iv administration of hPTH(1-34) showed more pulse-like PK compared with sc administration.

**Table 1 Pharmacokinetics parameters of hPTH(1-34)**

| | Tmax (min) | Cmax (ng/mL) | T1/2 (min) | AUC (ng/mL*min) |
|---|---|---|---|---|
| iv | - | - | 6.9 | 1066 |
| sc | 20 | 9.0 | 10.8 | 230 |

### Example 4 Difference in effects on bone turnover by caudal vein administration and subcutaneous administration of PTH(1-34) in aged OVX rats

PTH(1-34) was administered to aged OVX rats by caudal vein (iv) administration and subcutaneous (sc) administration, and difference in effects on bone turnover, which caused by difference in PTH(1-34) PK, were examined.

hPTH(1-34) was dissolved in 10 mM acetic acid solution, and then adjusted aliquoted to 10 nmol/mL using 25 mmol/L phosphate-citrate buffer, 100 mmol/L NaCl, 0.05% Tween 80 buffer (pH 5.0). The obtained solution was stored -80°C until use.

Female SD-IGS rats at 33 weeks of age (Charles River Japan, Inc.) were used, 60 of which were ovariectomized (OVX) to remove both ovaries and 8 of which were sham-operated. After 28 weeks, the OVX group was measured for bone density and subdivided into groups of 8 rats each such that the mean BMD of all groups were identical.

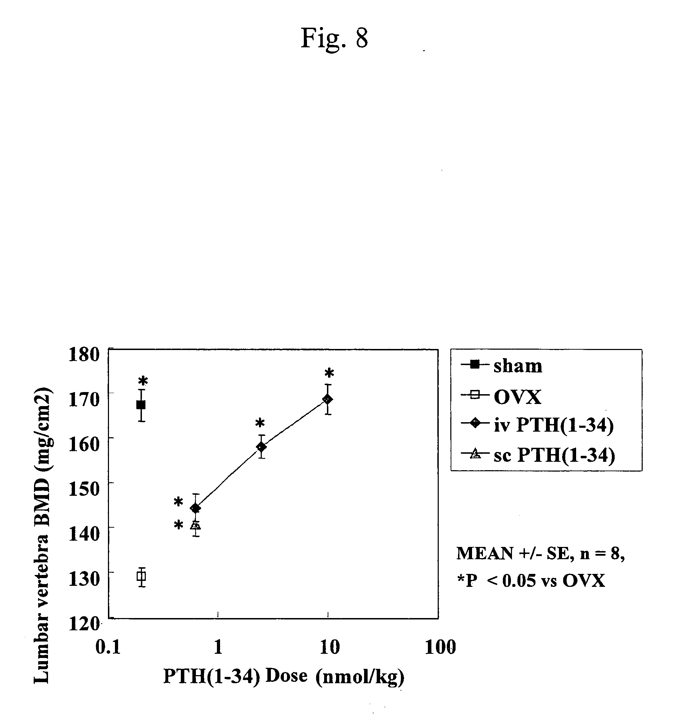

The stored hPTH(1-34) was diluted with phosphate-citrate buffer, and hPTH(1-34) was adjusted to 10, 2.5, and 0.625 nmol/ml. The buffer was administered to the sham group and the OVX group of 8 rats each. The diluted hPTH(1-34) was administered by iv administration to groups of 8 rats each at a dose of 1 ml/kg (10, 2.5, 0.625 nmol/kg) on a 5 times-a-week basis for 6 weeks. Further, 0.625 nmol/ml hPTH(1-34) was administered to one group at a dose of 1 ml/kg (0.625 nmol/kg) on a 5 times-a-week basis for 6 weeks. On the last day of administration, the rats were housed in metabolic cages and 24-hour urine was collected from each rat. On the following day, after the rats were euthanized by exsanguination under anesthesia, an autopsy was performed to collect blood, lumbar vertebrae and femurs. The urine and blood were taken into tubes and centrifuged to collect the respective supernatants, which were stored at -20°C until assayed for parameters. The lumbar vertebrae and right femurs were stored in 70% ethanol. The average bone density of the second to fifth lumbar vertebrae and the bone density of the right femur were measured using a dual X-ray bone mineral densitometer (DCS-600EX, ALOKA). Further, bone morphometry on the third lumbar vertebra was performed. The bone metabolism marker in serum and urine was measured. Figure 8 shows the results thereof.

As shown in Figure 8, the OVX group showed significant decrease in lumbar bone density over the sham group. Also, the group of hPTH(1-34) iv administration showed significant and dose-dependent increases in lumbar bone density over the OVX group. Further, the group of hPTH(1-34) sc administration showed a significant increase in bone density over the OVX group, and the increase was almost the same as the increase of bone density in iv administration at the same dose of 0.625 nmol/kg.

Deoxypyridinoline (DPD) in urine as bone resorption marker and tartrate-resistant acid phophatase form 5b (TRACP 5b) were measured. DPD was corrected by urine creatinine value (PPP/Cre). The results are shown in Figure 8.

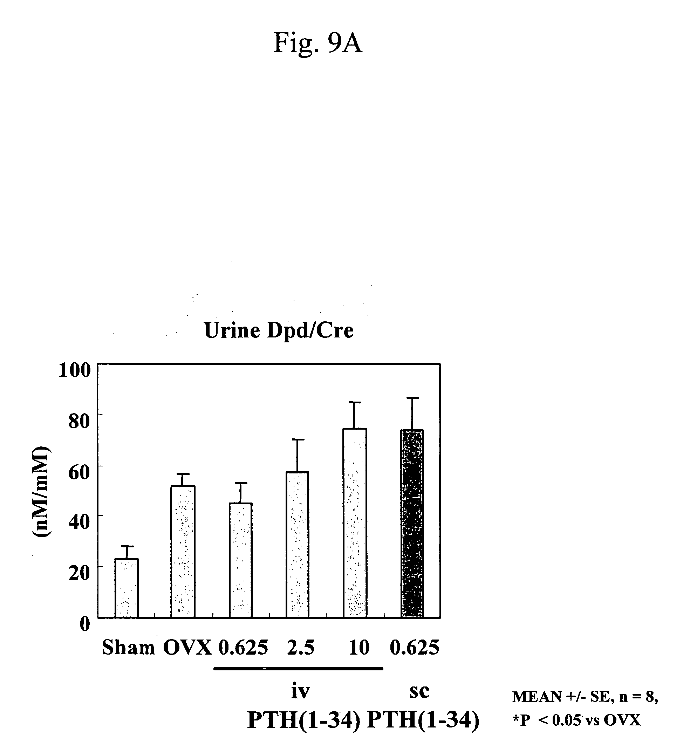

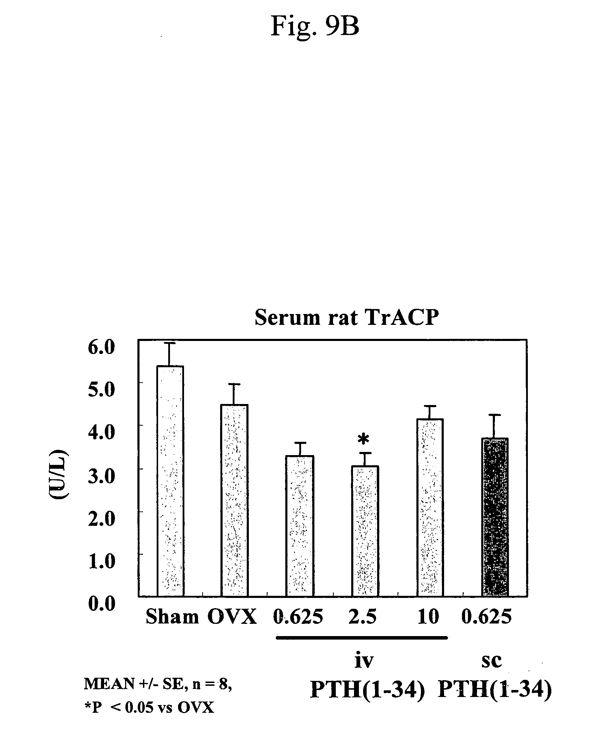

Regarding urine DPD, a decrease trend was observed in the iv administration group of 0.625 nmol/kg PTH(1-34), whereas an increase trend was observed in the sc administration group of 0.625 mnol/kg. Regarding blood TRACP 5b, a significant decrease was observed only in the iv administration group at 2.5 nmol/kg over the OVX group. The results are shown in Figures 9A and 9B.

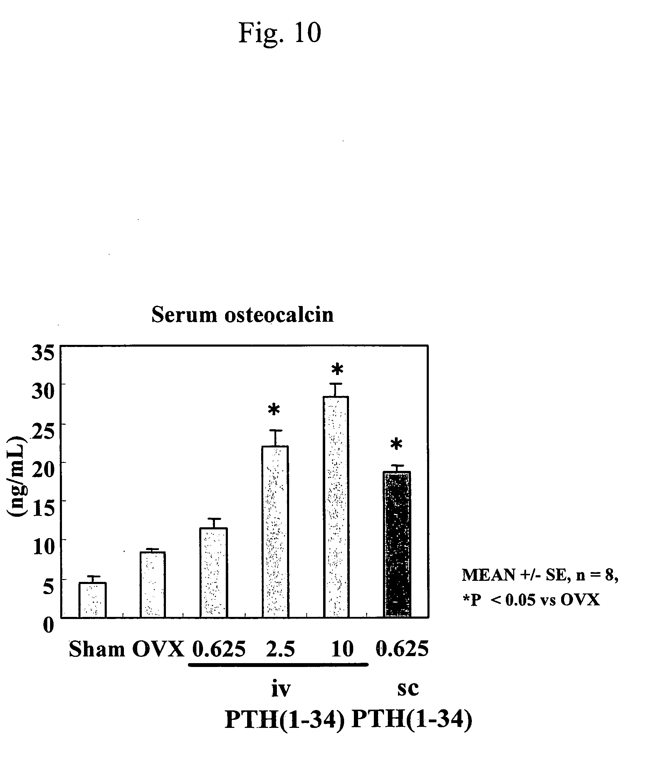

Osteocalcin (OC) in blood as bone formation marker was measured. The results are shown in Figure 10. Between the OVX group and the sham group, no significant change was observed. However, significant increases were observed in the iv administration groups at doses of 2.5 nmol/kg and 10 nmol/kg, and the sc administration group at a dose of 0.625 nmol/kg over the OVX group.

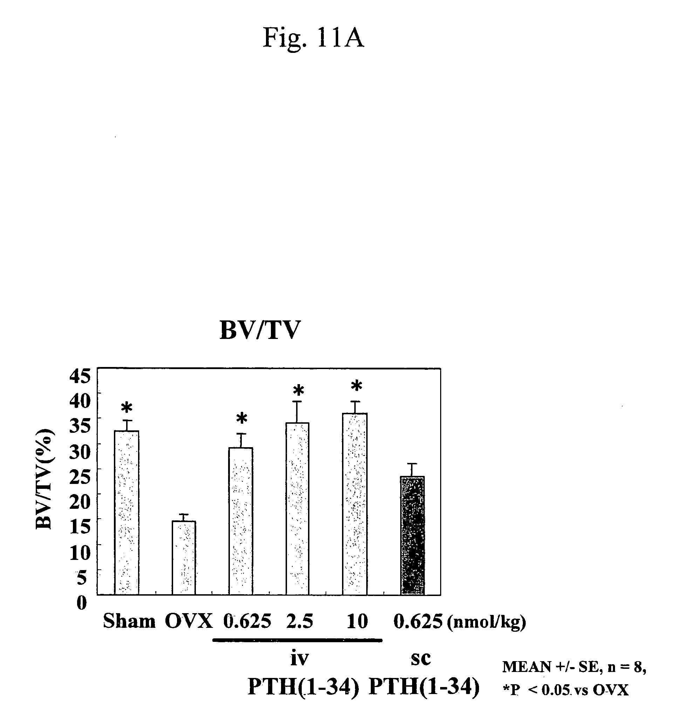

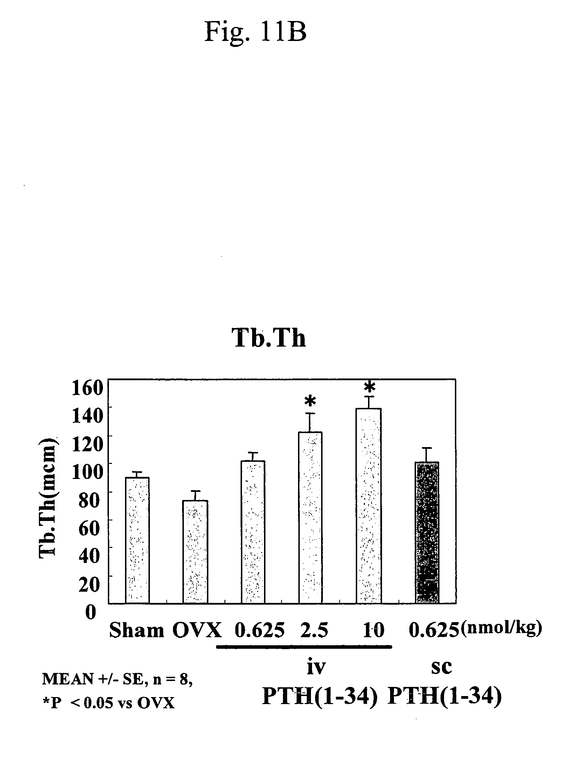

To investigate the influence of PTH(1-34) on bone turnover, bone morphometry was conducted. Among bone morphometry parameters, BV/TV is a parameter indicating bone mass. A significant and dose-dependent increase in BV/TV was observed in the PTH(1-34) iv administration group, whereas no significant increase was observed in the sc administration group (Figure 11A). Also, regarding parameter Tb.Th indicating bone mass, significant increases were observed in the iv administration groups at doses of 2.5 nmol/kg and 10 nmol/kg, whereas no significant increase was observed in the sc administration group (Figure 11B).

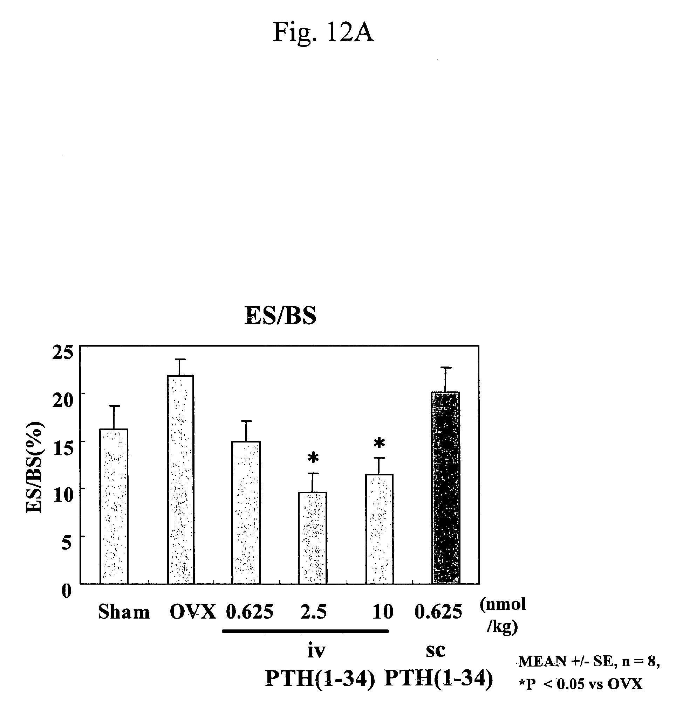

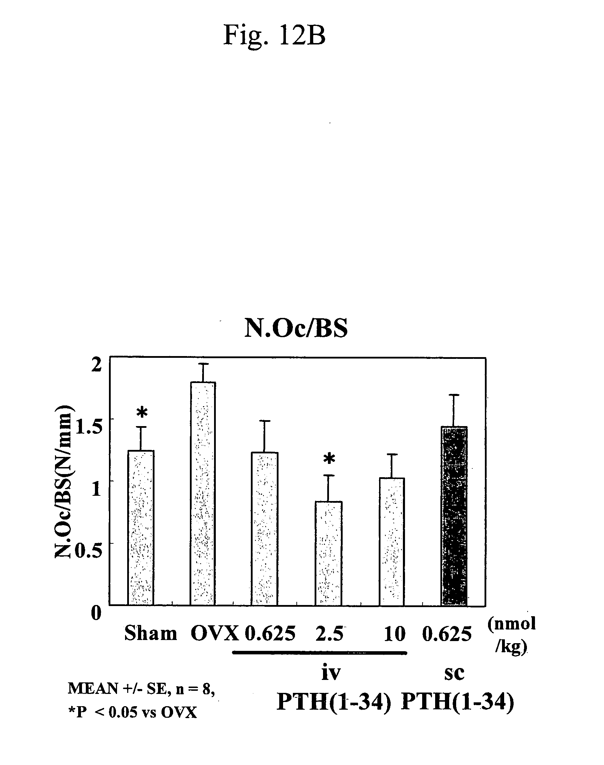

Regarding bone resorption parameter ES/BS, which is one of bone morphometry parameters, significant reductions from the level of the OVX group were observed in the PTH(1-34) iv administration groups at doses of 2.5 nmol/kg and 10 nmol/kg, whereas no significant decrease was observed in the sc administration group (Figure 12A). Regarding bone resorption parameter N.OcBS, a significant decrease was observed only in the PTH(1-34) iv administration group at a dose of 2.5 nmol/kg over the OVX group (Figure 12B).

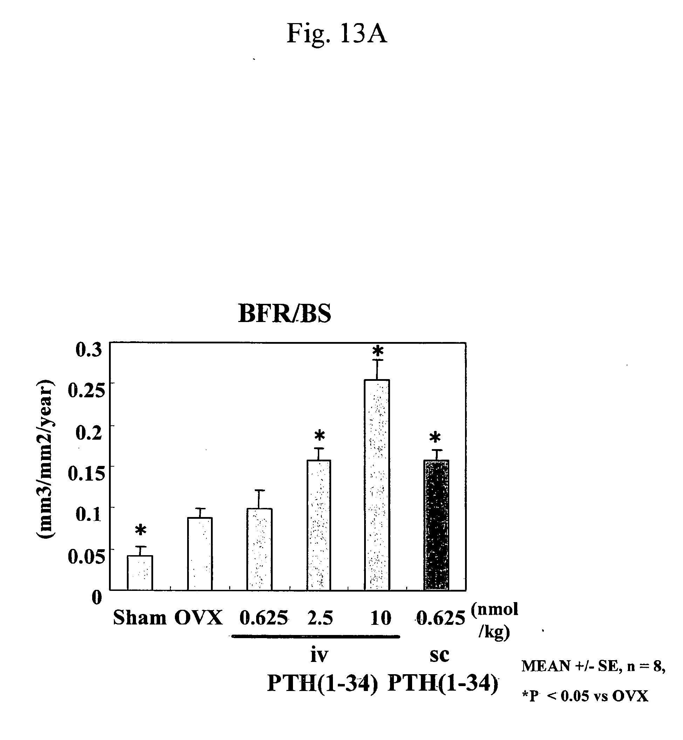

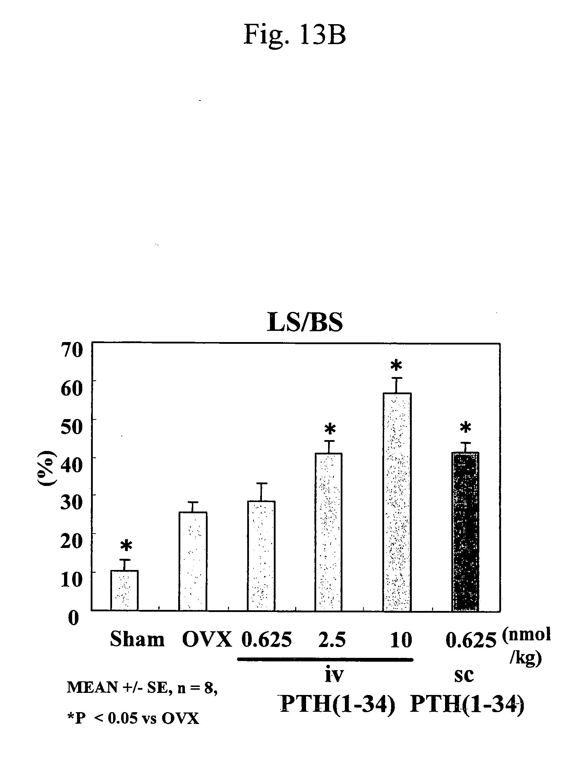

Regarding bone formation parameters BFR/BS and LS/BS, which is one of bone morphometry parameters, a significant increase was observed in the OVX group over the sham group. Significant increases were observed in the PTH(1-34) iv administration groups at doses of 2.5 nmol/kg and 10 nmol/kg and the PTH(1-34) sc administration group over the OVX group. Figures 13A and 13B show the measured parameters BFR/BS and LS/BS, respectively.

In view of the foregoing, it has been revealed that the iv and sc administrations of PTH(1-34) showed BMD increasing effects but they showed different effects in bone turnover. That is, the PTH(1-34) iv administration group increased bone formation marker and bone formation parameters of bone morphometry, while decreased bone resorption marker and bone resorption parameters of bone morphometry. This has indicated that the iv administration group inhibits bone resorption while promoting bone formation. On the other hand, although the PTH(1-34) sc administration group increased bone formation marker and bone formation parameters of bone morphometry, no changes in bone resorption parameters were observed. This has indicated that the sc administration group does not inhibit bone resorption.

All publications, patents, and patent applications cited herein are incorporated herein by reference in their entirety.

## Claims

1. A pharmaceutical composition for transmucosal administration containing hPTH or a derivative thereof.

2. The pharmaceutical composition for transmucosal administration according to claim 1, wherein the composition is a bone mass increasing agent.

3. The pharmaceutical composition for transmucosal administration according to claim 1, wherein the composition is a bone density increasing agent.

4. The pharmaceutical composition for transmucosal administration according to claim 1, wherein the composition is a therapeutic agent for osteoporosis.

5. The pharmaceutical composition for transmucosal administration according to claim 1, wherein the composition is a bone resorption inhibitor.

6. The pharmaceutical composition according to any one of claims 1 to 5, wherein the composition accelerates bone formation and inhibits bone resorption.

7. The pharmaceutical composition according to any one of claims 1 to 6, wherein the composition is a transnasal administration agent.

8. The pharmaceutical composition according to any one of claims 1 to 7, wherein hPTH is hPTH1-34.

9. The pharmaceutical composition according to any one of claims 1 to 8, wherein a dose of the composition per day is 250 µg to 1,000 µg.

10. The pharmaceutical composition according to any one of claims 1 to 8, wherein the composition is formulated such that a dose of the composition per day is 250 µg to 1,000 µg.

11. A pharmaceutical composition for transmucosal administration containing hPTH or a derivative thereof, wherein the composition is administered for purposes of increasing bone mass or bone density, and inhibiting an increase of risk of developing one or more symptoms selected from the group consisting of leg cramps, nausea, headache, and dizziness associated with hPTH administration.

12. A pharmaceutical composition for transmucosal administration containing hPTH or a derivative thereof, wherein the composition is administered for purposes of increasing bone mass or bone density, and inhibiting an increase of probability of developing one or more symptoms selected from the group consisting of leg cramps, nausea, headache, and dizziness associated with hPTH administration.

13. A pharmaceutical composition for transmucosal administration containing hPTH or a derivative thereof, wherein the composition is administered for purposes of increasing bone mass or bone density, and preventing a high frequency of developing one or more symptoms selected from the group consisting of leg cramps, nausea, headache, and dizziness associated with hPTH administration.

14. A pharmaceutical composition for transmucosal administration containing hPTH or a derivative thereof, wherein the composition is administered for purposes of maintaining an action of hPTH for bone mass increase or bone density increase, and reducing a risk of developing one or more symptoms selected from the group consisting of leg cramps, nausea, headache, and dizziness associated with hPTH administration.

15. A pharmaceutical composition for transmucosal administration containing hPTH or a derivative thereof, wherein the composition is administered for purposes of maintaining an action of hPTH for bone mass increase or bone density increase, and reducing a probability of developing one or more symptoms selected from the group consisting of leg cramps, nausea, headache, and dizziness associated with hPTH administration.

16. A pharmaceutical composition for transmucosal administration containing hPTH or a derivative thereof, wherein the composition is administered for purposes of maintaining an action of hPTH for bone mass increase or bone density increase, and reducing a frequency of developing one or more symptoms selected from the group consisting of leg cramps, nausea, headache, and dizziness associated with hPTH administration.

17. A pharmaceutical composition for transmucosal administration containing hPTH or a derivative thereof, wherein the composition is administered for a purpose of maintaining both functions of hPTH for bone formation and bone resorption inhibition.

18. The pharmaceutical composition according to any one of claims 11 to 17, wherein transmucosal administration is by a nasal mucosa.

19. The pharmaceutical composition according to any one of claims 11 to 18, wherein hPTH is hPTH1-34.

20. The pharmaceutical composition according to any one of claims 11 to 19, wherein a dose of the composition per day is 250 µg to 1,000 µg.

21. The pharmaceutical composition according to any one of claims 11 to 19, wherein the composition is formulated such that a dose of the composition per day is 250 µg to 1,000 µg.

22. A method for reducing a risk of developing one or more symptoms selected from the group consisting of leg cramps, nausea, headache, and dizziness associated with hPTH administration, the method comprising administering hPTH or a derivative thereof by transmucosal administration.

23. A method for reducing a probability of developing one or more symptoms selected from the group consisting of leg cramps, nausea, headache, and dizziness associated with hPTH administration, the method comprising administering hPTH or a derivative thereof by transmucosal administration.

24. A method for reducing a frequency of developing one or more symptoms selected from the group consisting of leg cramps, nausea, headache, and dizziness associated with hPTH administration, the method comprising administering hPTH or a derivative thereof by transmucosal administration.

25. The method according to any one of claims 22 to 24, wherein an action of hPTH for bone mass increase or bone density increase is maintained.

26. A method for maintaining both functions for bone formation and bone resorption inhibition, comprising administering hPTH or a derivative thereof by transmucosal administration.

27. The method according to any one of claims 22 to 26, wherein transmucosal administration is by a nasal mucosa.

28. A method for maintaining an inhibition function for bone resorption, comprising administering hPTH or a derivative thereof by an administration method having a shorter half life in blood in comparison with subcutaneous administration.

29. A method for maintaining bone formation function and bone resorption inhibition function, comprising administering hPTH or a derivative thereof by an administration method having a shorter half life in blood in comparison with subcutaneous administration.

30. The method according to any one of claims 22 to 29, wherein hPTH is hPTH1-34.

31. The method according to any one of claims 22 to 30, wherein a dose of the composition per day is 250 µg to 1,000 µg.
